Europäisches Patentamt

European Patent Office

Office européen des brevets

(19)

(11) Veröffentlichungsnummer: **0 002 695**
**B1**

(12) **EUROPÄISCHE PATENTSCHRIFT**

(45) Veröffentlichungstag der Patentschrift:
04.11.81

(21) Anmeldenummer: 78101589.6

(22) Anmeldetag. 07.12.78

(51) Int. Cl.³: **C 07 G 17/00,** C 12 P 13/00,
**A 01 N** 61/00 // C12R1/62

(54) Organisch-chemische Verbindung, Verfahren zu ihrer Herstellung, ihre Verwendung, sie enthaltende Mittel sowie Mikroorganismen.

(30) Priorität: 24.12.77 DE 2758008
11.02.78 DE 2805855

(43) Veröffentlichungstag der Anmeldung:
11.07.79 Patentblatt 79/14

(45) Bekanntmachung des Hinweises auf die Patenterteilung:
04.11.81 Patentblatt 81/44

(84) Benannte Vertragsstaaten:
BE CH DE FR GB IT NL

(56) Entgegenhaltungen:
**CHEMICAL ABSTRACTS, Vol. 72, 1970,
Columbus, Ohio, USA,
G. F. GAUZE et al.: »Production of
the antibiotic sibiromycin by
streptosporangium sibiricum«,
Seite 269, rechte Spalte,
Zusammenfassung Nr. 41 684f
CHEMICAL ABSTRACTS, Vol. 84, 1976,
Columbus, Ohio, USA,
A. TAMURA et al.: »Antibiotic
sporacuracin production«, Seite 365, rechte
Spalte, Zusammenfassung Nr. 87 957y**

(73) Patentinhaber: **BAYER AG, Zentralbereich Patente,
Marken und Lizenzen, D-5090 Leverkusen 1, Bayerwerk
(DE)**

(72) Erfinder: **Berg, Dieter, Dr., Claudiusweg 9,
D-5600 Wuppertal-1 (DE)**
Erfinder: **Frommer, Werner, Dr., Claudiusweg 17,
D-5600 Wuppertal-1 (DE)**
Erfinder: **Schmidt, Delf, Dr., Am Eckbusch 55b,
D-5600 Wuppertal-1 (DE)**
Erfinder: **Schmidt, Robert Rudolf, Dr., Hahnenweg 5,
D-5000 Köln 80 (DE)**
Erfinder: **Schäfer, Dietmar, Dr., Zum Lahnberg 3,
D-3550 Marburg 16 (DE)**

## Organisch-chemische Verbindung, Verfahren zu ihrer Herstellung, ihrer Verwendung, sie enthaltende Mittel sowie Mikroorganismen

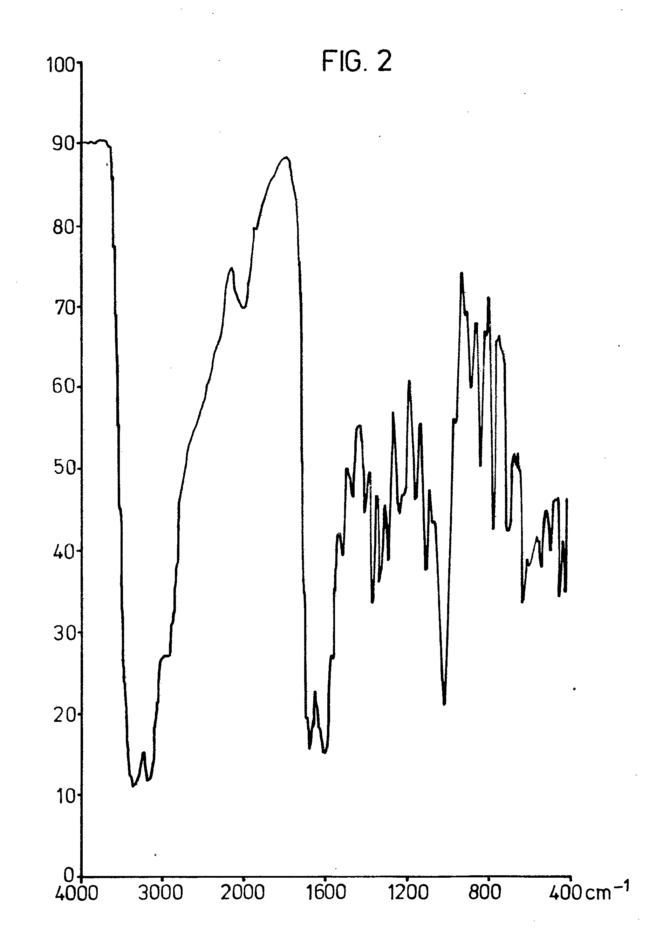

Die erfindungsgemäße Verbindung läßt sich durch die Summenformel $C_{10}H_{14}N_6O_3$ sowie das in Figur 2 wiedergegebene IR-KBr-Absorptionsspektrum (Abszisse: Wellenzahl in cm⁻¹, Ordinate: Extinktion), das charakteristische Banden bei den Wellenzahlen 3340, 3170, 2920, 2040, 1660, 1600, 1570, 1480, 1430, 1380, 1340, 1295, 1250, 1215, 1160, 1110, 1030, 890, 860, 790 und 720 cm⁻¹ aufweist, charakterisieren.

Des weiteren kann die Verbindung durch die folgenden Eigenschaften und Parameter beschrieben werden:

(1) Elementaranalyse (nach 4d Trocknen im Hochvakuum bei 100° C):

Berechnet:    C 45,11    H 5,30    N 31,57    O 18,03
Gefunden:    C 45,0    H 5,5    N 31,2    O 18,3

⁺) aus der Differenz berechnet.

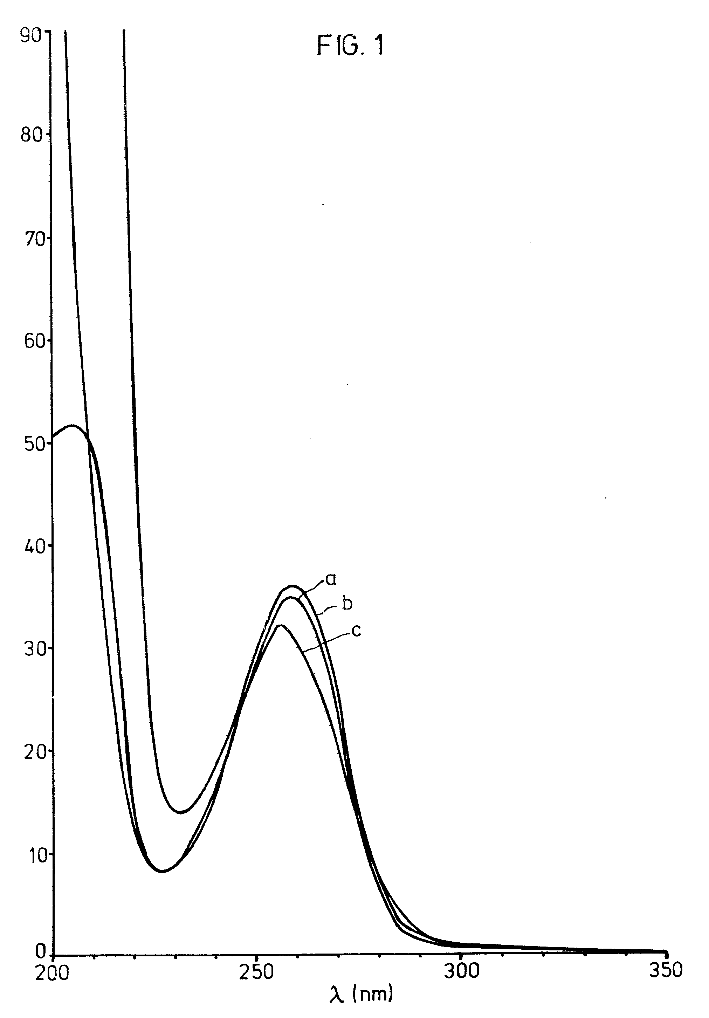

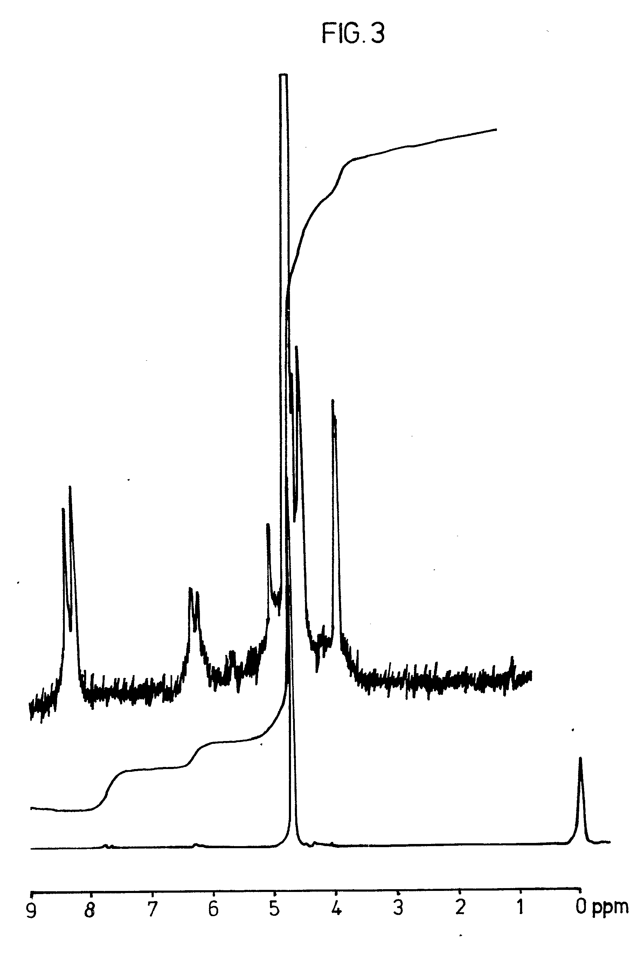

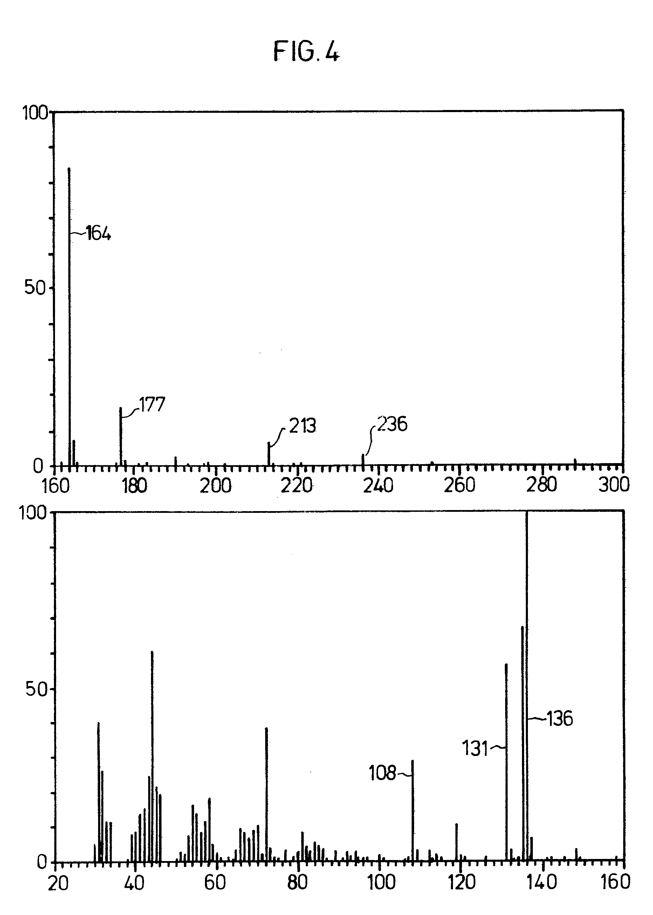

(2) Zersetzungspunkt:
Die Substanz bildet ein weißes amorphes Pulver mit einem Zersetzungspunkt von 134 – 135° C.
(3) Spezifische Drehung:
$[\alpha]_D^{20} = -10{,}7°$ (C = 1,0% in Methanol).
(4) Molekulargewichtsbestimmung:
Das Molekulargewicht wurde titrimetrisch zu 258 bestimmt im Feld-Desorptions-Massenspektrum wurde ein Quasi-Molekülion entsprechend einer Molmasse von 266 beobachtet.
(5) Ultraviolettabsorptionsspektrum:
Das UV-Spektrum der Verbindung wird in Figur 1 wiedergegeben (Abszisse: Wellenlänge in nm, Ordinate: Extinktion).
Es besitzt im ultravioletten Bereich ein charakteristisches Spektrum mit $\lambda_{max} = 259$ in $H_2O$ (Figur 1, Kurve b), $\lambda_{max} = 259$ in 0,1 n NaOH (Figur 1, Kurve a) und $\lambda_{max} = 257$ in 0,1 n HCl (Figur 1, Kurve c), in $H_2O$ ist $E_{1\,cm}^{1\%} = 401$ bei 259 nm, in 0,1 n NaOH ist $E_{1\,cm}^{1\%} = 341$ bei 259 nm, und in 0,1 n HCl ist $E_{1\,cm}^{1\%} = 337$ bei 257 nm.
(6) ¹H-60 MHz-Kernresonanzspektrum (gemäß Figur 3):
Das ¹H-NMR-Spektrum der Verbindung in $D_2O$ zeigt charakteristische Resonanzen bei bei $\delta = 8{,}37$, 8,25, 6,27 (Dublett) und 3,50 ppm (Dublett, breit).
(7) Massenspektrum (gemäß Figur 4):
Im Massenspektrum treten charakteristische Fragmentionen bei 236, 213, 177, 164, 136, 135, 131 und 108 auf.
(8) Die Verbindung ist leicht löslich in Wasser, wäßrigem Methanol und wäßrigem Äthanol, schwer löslich in wasserfreiem Methanol und wasserfreiem Äthanol, unlöslich in Äthylacetat, Butylacetat und Benzol.
(9) Die erfindungsgemäße Verbindung läßt sich sauer hydrolisieren. So werden beispielsweise bei 20 Min. langer Behandlung mit 6 N Salzsäure zwei Spaltprodukte aufgefunden. Das eine Spaltprodukt weist das charakteristische UV-Spektrum auf, das andere stellt einen Kohlenhydratanteil des Ausgangsproduktes dar.
(10) Die erfindungsgemäße Verbindung ist ein amorpher farbloser basischer Stoff. Da bei der sauren Hydrolyse ein Kohlenhydratanteil abgespalten wird, ist es leicht möglich, die intakte Verbindung bei der Dünnschichtchromatographie auf einer Kieselgelplatte mit Zuckerreagentien (z. B. Anisaldehyd-Schwefelsäure oder Thymol-Schwefelsäure) anzufärben. Einige Reaktionen, die zur Identifikation dienen, sind in Tabelle 1 aufgeführt.
(11) Die $R_f$-Werte der erfindungsgemäßen Verbindung auf neutralen Kieselgelplatten gibt Tabelle 2 wieder.
(12) Die antibiotische Wirkung der erfindungsgemäßen Verbindung ist gering. So kann mit 100 µg an E. coli keine Hemmung beobachtet werden. Die Hemmung an Staph. aureus ist mit 3 µg gering.

Tabelle 1

Versuche zur chemischen Anfärbung der Verbindung

| Nr. | Reagenz/Reaktion nach *) | Anfärbung |
|---|---|---|
| 1 | Ninhydrin | + |
| 2 | Morgan/Elson | + |
| 3 | 4-Dimethylaminobenzaldehyd | + |
| 4 | $KMnO_4$, alk. | + |
| 5 | $Thymol/H_2SO_4$ | + |
| 6 | $Anisaldehyd/H_2SO_4$ | + |
| 7 | Perjodat | + |
| 8 | 2',7'-Dichlorfluorescein | + |
| 9 | Jod | + |
| 10 | Bromkresolgrün | — |
| 11 | Rhodamin B | — |
| 12 | Molybdatophosphorsäure | — |
| 13 | Anilinphthalat | — |
| 14 | Tollen's Reagenz | — |
| 15 | Diphenylamin | — |
| 16 | 2,4-Dinitrophenylhydrazin | — |
| 17 | Eisen(III)-chlorid | — |

*) Die Reagentien wurden nach den üblichen Vorschriften (vgl. E. Stahl, Dünnschichtchromatographie, 2. Auflage, Springer-Verlag Berlin—Heidelberg—New York, 1967) angesetzt.

Tabelle 2

Dünnschichtchromatographische Charakterisierung der Verbindung auf Kieselgel 60, F. 254, Schichtdicke 0,25 mm (Merck)

| Fließmittelsystem (Volumenteile) | $R_f$-Werte |
|---|---|
| n-Butanol/Eisessig/$H_2O$ = 50/25/25 | 0,27 |
| Chloroform/Methanol/Eisessig = 90/8/2 | 0,05 |
| Chloroform/Methanol/$H_2O$ = 80/20/2,5 | 0,09 |
| Aceton | 0,04 |
| Chloroform/Methanol = 80/20 | 0,12 |
| Chloroform/Methanol = 90/10 | 0,03 |
| Chloroform | 0 |
| Diäthyläther | 0 |
| Chloroform/Methanol/Ammoniak = 20/3/0,5 | 0,04 |
| n-Butylacetat/n-Butanol/Eisessig/Phosphatpuffer pH 7 = 50/10/25/15 | 0,08 |
| i-Propanol/2 N Ammoniak/$H_2O$ = 7/1/2 | 0,40 |

Es wurde weiterhin gefunden, daß man die erfindungsgemäße Verbindung erhält, wenn man Mikroorganismen der Gattung Streptosporangium in einem Nährmedium, welches assimilierbare Kohlenstoff- und Stickstoffquellen sowie Mineralsalze enthält unter aeroben Bedingungen kultiviert und die Verbindung isoliert.

Im Hinblick auf die Gattung Streptosporangium muß es als überraschend bezeichnet werden, daß von ihr die erfindungsgemäße Verbindung gebildet wird, da nach Aussagen der Literatur [siehe z. B. J. Berdy in Advances in Applied Microbiology, Vol. 18, 1974, S. 309, Academic Press, New York] die Gattung Streptosporangium in bezug auf die Sekundärstoffbildung bei weitem nicht so ergiebig ist wie z. B. die nahe verwandte Gattung Streptomyces.

Zur Durchführung des erfindungsgemäßen Verfahrens können insbesondere die Streptosporangium-Stämme SS 237, (CBS 545.77), SS 243 (CBS 544.77), SS 248 (CBS 543.77) und SS 268 (CBS 542.77) Verwendung finden.

Diese Stämme gehören der Klasse der Schizomyceten, der Ordnung Actinomycetales, der Familie der Actinoplanaceae und der Gattung Streptosporangium an. Alle Stämme wurden aus Erde isoliert. Die Stämme haben die folgenden Merkmale:

Tabelle 3

| | Stamm-Nummer SS 237 | SS 243 | SS 248 | SS 268 |
|---|---|---|---|---|
| Gattung | Streptosporangium | Streptosporangium | Streptosporangium | Streptosporangium |
| Herkunft | Österreich Mittersill im Pinzgau; Humuserde | Schweden Anesät; Erde vom Rand eines Wasserlaufs | Persien Eiburgsgebirge; Erde von der Paßhöhe an der Straße Teheran—Amul | Dänemark nahe zur deutschen Grenze; Humuserde |
| Isolierungsdatum | 6. 2. 1974 | 8. 2. 1974 | 14. 2. 1974 | 21. 2. 1974 |
| Anreicherungsverfahren | Erdprobenausstrich auf Agarplatte | Erdprobenausstrich auf Agarplatte | Erdprobenausstrich auf Agarplatte | Erdprobenausstrich auf Agarplatte |
| Substratmycel | ca. 0,3 – 1 $\mu$ breit | ca. 0,3 – 0,9 $\mu$ breit | ca. 0,3 – 0,9 $\mu$ breit | ca. 0,3 – 0,9 $\mu$ breit |
| Luftmycel | meist etwa 0,8 – 1 $\mu$ breit, verzweigt septiert; weiß, schmutzig weiß, bei guter Sporangienbildung schwach rosa | meist etwa 0,8 – 1 $\mu$ breit, verzweigt, septiert; weiß | meist etwa 0,8 – 1 $\mu$ breit, verzweigt, septiert; weiß, bei guter Sporangienbildung rosa | meist etwa 0,8 – 1 $\mu$ breit, verzweigt, septiert; weiß |
| Sporangienform | kugelig | kugelig | kugelig | kugelig |
| Sporangiengröße (auf KEHE-Agar) | 3 – 15 $\mu$ meist 7 – 13 $\mu$ | 3 – 13 $\mu$ meist 6 – 11 $\mu$ | 3 – 16 $\mu$ meist 8 – 13 $\mu$ | 3 – 13 $\mu$ meist 6 – 10 $\mu$ |
| Sporenform | ellipsoid unbegeißelt | ellipsoid, z. T. auch kugelig unbegeißelt | ellipsoid unbegeißelt | ellipsoid, z. T. auch kugelig unbegeißelt |
| Sporengröße | meist 1 × 1,3 – 1,5 $\mu$ | etwa 0,8 – 1 × 1 – 1,5 $\mu$ | etwa 0,9 – 1,3 × 1 – 1,8 $\mu$, vereinzelt auch länger | meist 1 × 1,3 – 1,4 $\mu$, wenn kugelig 1 – 1,4 $\mu$ $\varnothing$ |

Tabelle 4

| | Stamm-Nummer SS 237 | SS 243 | SS 248 | SS 268 |
|---|---|---|---|---|
| Melaninbildung auf | CPC-Agar — Ty-Agar — Gelatine — | CPC-Agar — Ty — Agar — Gelatine — | CPC-Agar — Ty — Agar — Gelatine — | CPC-Agar — Ty-Agar — Gelatine — |
| Nitratreduktion | — | + | — | — |
| Gelatine-Verflüssigung | — | — | — | — |
| Milch-Peptonisierung | + (gering) | + (gering) | + | + |
| Tyrosin-Auflösung | — | + | — | — |
| Wachstum bei | | | | |
| 15°C | + | + | + | + |
| 20°C | + | + | + | : |
| 27°C | + | + + | + | + + |
| 32°C | + | + | + + | + |
| 37°C | + | + | + | + |
| 42°C | — | — | — | — |
| Stärke-Hydrolyse | + + | + | + + | + |
| L-Arabinose | + + (!) | + + | + + (!) | + + (!) |
| D-Fructose | + + (!) | + + (!) | + + (!) | + + (!) |
| D-Glucose | + + (!) | + + | + + | + + (!) |
| i-Inosit | — | — | + + | — |
| Lactose | — | — | + | + |
| D-Mannit | + + (!) | + + (!) | + + (!) | + + (!) |
| Raffinose | — | — | + | — |
| L-Rhamnose | + + (!) | + | + + (!) | + + (!) |
| Saccharose | + + (!) | + + (!) | + + (!) | + + (!) |
| D-Xylose | + + (!) | — | + + (!) | + + (!) |

5

Tabelle 5

| | Stamm-Nummer SS 237 | SS 243 | SS 248 | SS 268 |
|---|---|---|---|---|
| Haferflocken-Hefe-Agar (HaH) | W gut<br>SM bräunlich orange und braun<br>Lm +, überwiegend als dünner reifartiger Belag; weiß und schmutzig gelblich<br>SP braun in der nächsten Umgebung des Mycels<br>RK ca. 10 mm ∅; Oberfläche wulstig und bucklig; Rand gelappt, scharf gegen den Nährboden abgesetzt; SM am Kolonierand orange, nach dem Zentrum hin orangebraun und braun | W gut<br>SM kräftig orangerot<br>LM —<br><br><br>SP —<br><br>RK ca. 10 mm ∅; Oberfläche nach schmaler flacher Randzone bucklig und gefurcht, Rand gelappt scharf gegen den Nährboden abgesetzt; SM kräftig orangerot | W gut<br>SM orangerot<br>LM —<br><br><br>SP —<br><br>RK ca. 8 mm ∅; Oberfläche bucklig; schmale, flache transparente Randzone; SM kräftig orange | W gut<br>SM hell orangerot<br>LM + (wenig), weiß<br><br><br>SP —<br><br>RK ca. 15 mm ∅, kompakt; Oberfläche mit radial verlaufenden Rinnen, konvexe Sektoren mit konzentrischen Rillen Koloniemitte wulstig; Rand gelappt, scharf gegen den Nährboden abgesetzt; SM kräftig orangerot LM + nahe dem Rand |
| Hefe-Stärke-Agar (E) | W gut<br>SM rötlich braun bis rötlich dunkelbraun<br>LM + + als dünner reifartiger Belag; Sporangien +<br>SP — bis schwach gelbbraun<br>RK ca. 10 mm ∅, stark in die Höhe gewachsen; Oberfläche schuppig; Rand gelappt, scharf gegen den Nährboden abgesetzt; SM rötlich dunkelbraun | W mäßig<br>SM rötlich gelbbraun<br><br>LM —<br><br>SP —<br>RK ca. 10 mm ∅, mit radialen Rinnen, Sektoren konzentrisch gestuft; Rand gelappt, scharf gegen den Nährboden abgesetzt; SM bräunlich gelb mit orangener Tönung | W gut<br>SM hell orangerot<br><br>LM + +, überwiegend als dünner reifartiger Belag<br>SP —<br>RK ca. 10 mm ∅, stark in die Höhe gewachsen; Oberfläche schuppig-warzig; Rand gelappt, scharf gegen den Nährboden abgesetzt; SM orange | W gut<br>SM gelbbraun<br><br>LM —<br><br>SP —<br>RK ca. 12 mm ∅, mit radialen Rinnen, Sektoren konzentrisch gestuft und schuppig; Rand gelappt, scharf gegen den Nährboden abgesetzt; SM braungelb mit schwach orangener Tönung |

0 002 695

Fortsetzung

| Stamm-Nummer | SS 237 | SS 243 | SS 248 | SS 268 |
|---|---|---|---|---|
| Casamino-Pepton-Czapek-Agar (CPC) | W mäßig bis gut<br>SM gelborange<br><br>LM —<br><br>SP —<br>RK ca. 7 mm ⌀, stark in die Höhe gewachsen; Oberfläche schuppig-warzig; Rand gelappt, scharf gegen den Nährboden abgesetzt; SM gelborange | W mäßig<br>SM orange und blaß ocker<br><br>LM —<br><br>SP —<br>RK ca. 5 mm ⌀; Oberfläche bucklig, beginnende Aufschuppung; Rand gelappt, scharf gegen den Nährboden abgesetzt SM gelborange | W mäßig bis gut<br>SM orange<br><br>LM + +, sehr dünner, reifartiger Belag,<br>SP —<br>RK ca. 5 mm ⌀; wie bei SS 243; Oberfläche leicht bereift durch schwache LM-Bildung | W mäßig bis gut<br>SM bräunlich gelb, z. T. blaß orange<br>LM —<br><br>SP —<br>RK ca. 8 mm ⌀, mit radialen Rinnen, Sektoren konzentrisch gerillt und gestuft; Rand gelappt, scharf gegen den Nährboden abgesetzt; SM bräunlich gelb mit schwach orangener Tönung |
| NO₃-Agar | W gut<br>SM schwarzbraun<br>LM —<br>SP —<br>Nitratreduktion — | W gut<br>SM blaß braunorange<br>LM —<br>SP —<br>Nitratreduktion + | W gut<br>SM hell orangebraun<br>LM —<br>SP —<br>Nitratreduktion — | W gut<br>SM braunorange<br>LM —<br>SP —<br>Nitratreduktion — |
| Magermilch-Agar (Ca) | W mäßig bis gut<br>SM rötlich braun<br>LM —<br><br>Milch geringfügig peptonisiert | W mäßig<br>SM orange<br>LM —<br>SP —<br>Milch geringfügig peptonisiert | W mäßig bis gut<br>SM hell bräunlich rot<br>LM —<br>SP —<br>Milch z. T. peptonisiert | W gut<br>SM orangerot<br>LM —<br>SP —<br>Milch z. T. peptonisiert |
| Tyrosin-Agar (Ty) | W mäßig<br>SM braun<br>LM —<br>SP —<br>Kristallauflösung — | W gering<br>SM gelblich braun<br>LM —<br>SP —<br>Kristallauflösung + | W gut bis mäßig<br>SM gelbbraun<br>LM —<br>SP —<br>Kristallauflösung — | W mäßig bis gut<br>SM rötlich braun<br>LM —<br>SP —<br>Kristallauflösung — |
| Czapek-Agar (Cz) | W gering bis mäßig<br>SM braun<br>LM —<br>SP schwach geblich braun | W gering<br>SM blaß bräunlich gelb<br>LM --<br>SP — | W mäßig<br>SM gelblich braun<br>LM —<br>SP — | W gering<br>SM blaß braun<br>LM —<br>SP — |

0 002 695

Fortsetzung

| Stamm-Nummer | | | |
|---|---|---|---|
| SS 237 | SS 243 | SS 248 | SS 268 |
| Erd-Glucose-Hefe-Agar (EGH) | | | |
| W gering bis mäßig | W gering bis mäßig | W gering bis mäßig | W gering bis mäßig |
| SM braun bis dunkelbraun | SM braun | SM braun | SM braun |
| LM + +, weiß | LM +, wenig | LM + +, weiß | LM +, wenig |
| Sporangien + + | Sporangien + | Sporangien + + | Sporangien + |
| Künstliche Erddekokt Hefe-Extrakt-Agar (KEHE) | | | |
| W gering | W gering | W gering | W gering |
| SM blaß orangebraun und blaß braun | SM braunorange | SM braunorange | SM blaß orangebraun |
| LM +, reifartig, weiß und schwach rosa | LM +, reifartig, weiß | LM +, reifartig, weiß | LM +, wenig; weiß |
| Sporangien + + | Sporangien + + | Sporangien + + | Sporangien + |
| SP − | SP − | SP − | SP − |

0 002 695

Erläuterungen zu den Tabellen

W = Wachstum
SM = Substratmycel
LM = Luftmycel
SP = substratverfärbender Farbstoff
RK = Riesenkolonie

Beim Test auf Verwertbarkeit verschiedener Kohlenstoffquellen bedeuten

+ + Wachstum so gut wie auf Mineralsalz-Vitamin-Agar mit Glucose oder besser
+ Wachstum besser als auf reinem Mineralsalz-Vitamin-Agar, aber geringer als auf MSV-Agar mit Glucose
– Wachstum wie auf reinem MSV-Agar oder geringer
(!) Wachstum gut

Die Stämme wurden bei Zimmertemperatur und diffusem Tageslicht kultiviert. Nach vier- bis sechswöchigem Wachstum wurden die Kulturmerkmale auf den einzelnen Nährmedien protokolliert. Die Angaben für die Riesenkolonien beziehen sich auf eine Wachstumsdauer von 6 Wochen.

Kulturmedien:

Erd-Glucose-Hefe-Agar (EGH): Erddekokt 250 ml; Glucose 0,5 g; Hefeextrakt 0,5 g; Agar 15 g; Leitungswasser 750 ml.

Zur Zusammensetzung der übrigen Nährmedien siehe SCHÄFER, D.: »Beiträge zur Klassifizierung und Taxonomie der Actinoplanaceen«, Dissertation, Marburg 1973 (insbesondere S. 42 f.).
Für den Test auf Verwertbarkeit verschiedener C-Quellen wurde der als Grundmedium dienende Mineralsalz-Agar mit einer Vitaminlösung angereichert, da die vier Stämme auf dem einfachen Medium nur geringes Wachstum zeigten.

Vitaminlösung:
Thiamin 100 mg; Biotin 0,1 mg; Nicotinsäureamid 100 mg; Inosit 1 g; Wasser 100 ml.

Pro Liter Mineralsalzagar wurde 1 ml dieser Vitaminlösung zugesetzt.
Die Anreicherung und Isolierung der Stämme erfolgten nach den üblichen Methoden (siehe SCHÄFER, 1973) durch Erdprobenausstrich auf Petrischalen, vier- bis sechswöchige Bebrütung und Abimpfung einzelner Sporangien.
Alle vier Stämme zeichnen sich durch kugelige, am Luftmycel entstehende Sporangien mit unbegeißelten Sporangiosporen aus. Aufgrund dieser morpholigischen Merkmale gehören sie eindeutig zur Gattung Streptosporangium.
Wie die Tabellen zeigen, sind die Stämme untereinander nicht gleich.
Das erfindungsgemäße Verfahren kann mit Hilfe fester, halbfester oder flüssiger Nährmedien durchgeführt werden. Bevorzugt werden wäßrig-flüssige Nährmedien verwendet.
Die Beimpfung der Nährmedien erfolgt nach allgemein üblichen Methoden, z. B. über Schrägröhrchen oder Kolbenkulturen.
Die Kultur erfolgt unter aeroben Bedingungen und kann gemäß den allgemein üblichen Methoden wie unter Verwendung von Schüttelkulturen z. B. in Schüttelkolben, von luftbewegten Kulturen oder von Submerskulturen durchgeführt werden. Bevorzugt erfolgt die Kultivierung im aeroben Submersverfahren in belüfteten Fermentern, z. B. in üblichen Submersfermentationstanks. Es ist möglich, die Kultur kontinuierlich oder diskontinuierlich durchzuführen. Vorzugsweise wird diskontinuierlich gearbeitet.
Die Kultur kann in allen Nährmedien durchgeführt werden, welche bekannterweise zur Kultivierung von Mikroorganismen der Ordnung Actinomycetales verwendet werden. Das Nährmedium muß eine oder mehrere assimilierbare Kohlenstoffquellen und Stickstoffquellen sowie Mineralsalze enthalten, wobei diese Produkte in Form von definierten Einzelbestandteilen, aber auch in Form von komplexen Gemischen, wie sie insbesondere biologische Produkte verschiedenen Ursprungs darstellen, vorliegen können. Als Kohlenstoffquellen kommen alle üblichen Kohlenstoffquellen infrage. Beispielsweise seien Stärke, Melasse, Molkepulver, Dextrin, Zucker, wie Saccharose, Maltose, Glucose, Lactose, Sorbit und Glycerin genannt. Als Stickstoffquellen kommen alle üblichen organischen und anorganischen Stickstoffquellen infrage. Beispielsweise seien Sojabohnenmehl, Baumwollsamenmehl, Linsenmehl, Erbsenmehl, lösliche und unlösliche pflanzliche Proteine, Maisquellwasser, Hefeextrakt, Peptone und Fleischextrakt sowie Ammoniumsalze und Nitrate, z. B. $NH_4Cl$, $(NH_4)_2SO_4$, $NaNO_3$ und $KNO_3$ aufgeführt.

Bei der Durchführung des Verfahrens kann es günstig sein, zu Beginn der Kultivierung nur relativ geringe Konzentrationen der löslichen Nährlösungsbestandteile zu verwenden und dann im Laufe der ersten 3 Kultivierungstage durch häufigere Zusätze diese Bestandteile in Form steriler, relativ konzentrierter Lösung dem Kulturansatz fraktioniert zuzufüttern. Der pH-Wert der wachsenden Kulturen sollte vorzugsweise zwischen etwa 6 und etwa 8, insbesondere zwischen 6,5 und 7,5 gehalten werden. Ein zu starker pH-Abfall in den sauren Berich kann durch Zusätze einer organischen oder anorganischen Base, vorzugsweise von CaCO$_3$ vermieden werden. Wie in der Fermentationstechnologie üblich, kann auch eine automatische pH-Regulierung durchgeführt werden.

Es ist zweckmäßig, sicherzustellen, daß die Mikroorganismen ausreichend mit Sauerstoff sowie den Nährstoffen in Kontakt gebracht werden. Dies kann nach den allgemein üblichen Methoden wie Schütteln und Rühren erfolgen.

Die Züchtungstemperatur kann zwischen etwa 20 und etwa 40°C, vorzugsweise zwischen 24 und 35°C liegen, besonders bevorzugt liegt sie bei etwa 28°C. Die Dauer der Züchtung kann stark variiert werden, wobei z. B. die Zusammensetzung des Nährmediums und die Züchtungstemperatur eine Rolle spielen.

Es hat sich herausgestellt, daß die Menge des sich in der Kulturbrühe anreichernden erfindungsgemäßen Verbindung im allgemeinen ihr Maximum etwa 2 bis 12, vorzugsweise 5 bis 8 Tage nach Züchtungsbeginn erreicht.

Wie allgemein bei mikrobiologischen Verfahren sollten Fremdinfektionen der Kulturmedien vermieden werden.

Falls bei der Kultivierung in unerwünschter Menge Schaum entsteht, können in üblichen chemischen Schaumdämpfungsmittel, z. B. flüssige Fette und Öle, Öl-Wasser-Emulsionen, Paraffine, höhere Alkohole, wie Octcdecanol, Siliconöle, Polyoxyäthylen- bzw. Polyoxypropylenverbindungen zugesetzt werden. Schaum kann auch mit Hilfe der üblichen mechanischen Vorrichtungen (welche z. B. Zentrifugalkräfte benutzen) gedämpft oder beseitigt werden.

Die erfindungsgemäße Verbindung kann aus dem Kulturmedium nach allgemein üblichen physikalisch-chemischen Methoden isoliert werden. Die Isolierung kann z. B. gemäß den üblichen Extraktionsverfahren, Fällungsverfahren und/oder Chromatographieverfahren erfolgen. Das isolierte Herbizid kann mit Hilfe der genannten Methoden auch feingereinigt werden. Für viele Fälle ist jedoch eine Feinreinigung nicht erforderlich, da die gegebenenfalls vorhandenen Verunreinigungen die Wirksamkeit der Verbindung als Herbizid nicht nachteilig beeinflussen. Bei allen Isolierungs- und Reinigungsoperationen ist darauf zu achten, daß pH-Werte oberhalb 3,0 eingehalten werden. Zur Erhöhung des pH-Wertes können anorganische und organische Basen verwendet werden, z. B. Ammoniak, Alkali- und Erdalkalihydroxide, Alkali- und Erdalkalicarbonate und Hydrogencarbonate, z. B. KOH, NaOH, Na$_2$CO$_3$, NaHCO$_3$, CaCO$_3$, Trialkylamine wie Triäthylamin, Morpholin und Pyridin. Um bei den obengenannten Isolierungs- und Reinigungsmethoden die Fraktionen herauszufinden, in welchen die erfindungsgemäße Verbindung in höchster Konzentration bzw. Reinheit vorliegt, können die üblichen physikalisch-chemischen Methoden, z. B. Messen der UV-Bande bei 259 nm, der R$_f$-Werte oder vorzugsweise die Untersuchung der herbiziden Aktivität herangezogen werden.

Die Isolierung und Reinigung der erfindungsgemäßen Verbindung kann z. B. im Falle, daß ein flüssiges wäßriges Nährmedium verwendet wird, wie folgt vorgenommen werden:

Nach seiner Anreicherung im Kulturüberstand werden Kulturfiltrat und Mycel mit üblichen Methoden separiert (z. B. Zentrifugation).

Aus dem Kulturfiltrat kann die erfindungsgemäße Verbindung mit Hilfe von üblichen Extraktionsverfahren, Fällungsverfahren und/oder Chromatographieverfahren isoliert und gegebenenfalls gereinigt werden. Die Chromatographie kann in Form der Säulenchromatographie durchgeführt werden. Als Adsorptionsmittel können die üblichen anorganischen oder organischen Adsorptionsmittel eingesetzt werden, wie z. B. Aluminiumoxid, Kieselgel, Magnesiumsilikat, Aktivkohle, Cellulose, Cellulosederivate, Kunstharze wie Polyamide, Derivate von Polyamiden, z. B. acetyliertes Polyamid oder Dextrangele. Als Laufmittel können die verschiedensten Lösungsmittel oder Lösungsmittelgemische verwendet werden, in welchen die erfindungsgemäße Verbindung löslich ist. Bevorzugt wird Wasser oder ein Gemisch aus Wasser und Methanol oder aus Wasser und Äthanol (z. B. 1 : 1 Volumenteile) eingesetzt.

Bevorzugt werden zur Isolierung der erfindungsgemäßen Verbindung Chromatographie-Verfahren verwendet, z. B. unspezifische Adsorption an hydrophobe Sorbentien oder andererseits Ionenaustauschchromatographie. Dies sind die von der Reinigung wasserlöslicher basischer Naturstoffe her bekannte Methoden.

Für die komerzielle Herstellung des erfindungsgemäßen Herbizids bevorzugt man die Gewinnung durch Adsorption und anschließende Desorption an einem hydrophoben Trägerharz (z. B., Lewapol; ein hydrophobes Trägerharz der Bayer AG). Die Desorption kann z. B. mit kurzkettigen aliphatischen Alkoholen durchgeführt werden, bevorzugt Methanol oder Äthanol. Das Desorptionsmittel sollte mit Wasser mischbar sein, da die Löslichkeit der Verbindung in wasserfreien Alkoholen gering ist.

Weiterhin kann eine Ionenaustauschchromatographie durchgeführt werden, vorzugsweise an Ionenaustauschern vom Phosphorsäuretyp (z. B. Phosphocellulose) oder vom Sulfonsäuretyp (z. B. Dowex® 50-Typen; Sulfonsäureharze der Dow Chem. Corp., Midland, Michigan), aber auch schwächer saure Austauscher vom Carbonsäuretyp (wie z. B. Lewatit® CNP/LF; schwach saurer Austauscher der Bayer AG) oder Amberlite /RC-50 schwach saurer Austauscher der Fa. Röhm u. Haas IRC-50) können eingesetzt werden. Der Ionenaustausch kann mit Hilfe der Gradientenelution durchgeführt werden, wobei sowohl reine pH-Gradienten, reine Salzgradienten, als auch gemischte pH-Salz-Gradienten erfolgreich eingesetzt werden können. Der Ionenaustausch kann auch im »batch«-Verfahren durchgeführt werden, wobei zur Desorption vorzugsweise höhere Salzkonzentrationen in Wasser verwendet werden (z. B. 1 M Ammoniumformiat in Wasser), aber auch durch Erhöhung des pH-Wertes (z. B. auf pH 10) kann die Desorption erreicht werden.

Eine derart vorgereinigte Fraktion kann wiederum mit den üblichen Methoden gereinigt werden. Bevorzugt kann hier die Gelddiffusionschromatographie eingesetzt werden. Die Chromatographie an hochvernetzten Polyacrylamidgelen(beispielsweise an Biogel® P-2) verläuft erfolgreich, aber auch mit Hilfe von Dextranen (z. B. Sephadex® G-10) kann die Isolierung der erfindungsgemäßen Verbindung durchgeführt werden. Vorzugsweise wird hier in Gegenwart einwertiger Ionen gearbeitet, um unspezifische Wechselwirkungen zwischen den Trägermaterialien und der chromatographisch zu entwickelnden Verbindung zu vermeiden. Ein derartig gewonnenes Produkt ist im allgemeinen reiner als 80%.

Die Reinsubstanz der erfindungsgemäßen Verbindung kann aus einer solchen Fraktion mit den üblichen biochemischen Methoden gewonnen werden. Von den bereits genannten Adsorptionsmitteln kann neben anderen vor allem Cellulose erfolgreich eingesetzt werden. Als Fließmittel kommt z. B. Chloroform-Methanol (so etwa 4/1 Volumenteile) mit Erfolg zur Anwendung.

Aus seinen Lösungen kann die Verbindung nach den üblichen Methoden, z. B. Verdampfen des Lösungsmittels, Gefriertrocknung usw. erhalten werden.

Die neuen Streptosporangium-Stämme mit den Laborbezeichnungen SS 237, SS 243, SS 248, SS 268 wurden am 28. November 1977 unter den folgenden Nummern beim Centralbureau vor Schimmelcultures, Baarn, Niederlande hinterlegt:

SS 237    CBS 545.77
SS 243    CBS 544.77
SS 248    CBS 543.77
SS 268    CBS 542.77

Die erfindungsgemäße Verbindung beeinflußt das Pflanzenwachstum und kann deshalb als defoliant, Desiccant, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob der erfindungsgemäße Stoff als totales oder selektives Herbizid wirkt, hängt im wesentlichen von der angewendeten Menge ab.

Die erfindungsgemäße Verbindung kann z. B. bei den folgenden Pflanzen verwendet werden:

Dikotyle Unkräuter der Gattungen:
Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sebania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:
Gossypium, Glycine, Beta, Daucus, Phaseolus, Pisum, Solanum, Linium, Ipomoea, Vicia, Nicotiana, Lycopersion, Arachis, Brassica, Lactuca, Cucumis, Cuburbita.

Monokotyle Unkräuter der Gattungen:
Echinochloa, Setaria, Panicum, Digitaria, Phleum, Poa, Eleusine, Brachiaria, Lolium, Bromus, Avena, Cyperus, Sorghum, Agropyron, Cynodon, Monocharia, Fimbristylis, Sagittaria, Eleocharis, Scirpus, Paspalum, Ischaemum, Spenoclea, Dactyloctenium, Agrostis, Alopecurus, Apera.

Monokotyle Kulturen der Gattungen:
Oryza, Zea, Triticum, Hordeum, Avena, Secale, Sorghum, Panicum, Saccharum, Ananas, Asparagus, Allium.

Die Verwendung des erfindungsgemäßen Wirkstoffs ist jedoch keineswegs auf diese Gattungen Beschränkt, sondern erstreckt sich in gleicher Weise auf andere Pflanzen.

Die Verbindung eignet sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Der Wirkstoff kann in die üblichen Formulierungen übergeführt werden wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä. sowie ULV-Kalt- und Warmnebel-Formulierungen.

Diese Formulierungen werden in bekannter Weise hergestellt, z. B. durch Vermischen des Wirkstoffs mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate: als feste Trägerstoffe für Granulate:
gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z. B. Alkylaryl-polyglykol-äther, Alkylsulfonate, Alkylsulfate, Arylsulfonate sowie Eiweißhydrolysate; als Dispergiermittel: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe, wie anorganische Pigmente, z. B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäße Verbindung kann als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Der Wirkstoff kann als solcher, in Form seiner Formulierungen oder der daraus durch weiteres Verdünnen bereiteten Anwendungsformen, wie gebrauchsfertige Lösungen, Emulsionen, Suspensionen, Pulver, Pasten und Granulate angewandt werden. Die Anwendung geschieht in üblicher Weise, z. B. durch Gießen, Spritzen, Sprühen, Stäuben, Streuen, Trockenbeizen, Feuchtbeizen, Naßbeizen, Schlämmbeizen oder Inkrustieren.

Die erfindungsgemäße Verbindung kann sowohl nach als auch insbesondere vor Auflaufen der Pflanzen appliziert werden.

Die aufgewandte Wirkstoffmenge kann in größeren Bereichen Schwanken. Sie hängt im wesentlichen von der Art des gewünschten Effekts ab. Im allgemeinen liegen die Aufwendungen zwischen 0,1 und 40 kg Wirkstoff pro ha, vorzugsweise zwischen 0,1 und 10 kg/ha.

Es ist bereits bekannt geworden, daß eine Spezies von Streptomyces (S. Saganonens) zwei herbizid wirksame Komponenten, die Herbizidine A und B bildet [Journal of Antibiotics 29 (9), 863 – 869 (1976); ibid 29 (9), 870 – 875 (1976)].

## Beispiel 1

Beimpft man mit je 4 ml einer Vorkultur (gewonnen in derselben Nährlösung, beimpft mit einer Schrägröhrchenkultur) des Stammes SS 237 1-Ltr.-Erlenmeyerkolben, die 120 ml einer Nährlösung der Zusammensetzung 0,5% $(NH_4)_2SO_4$, 0,3% $KNO_3$, 0,4% Sojamehl, 0,4% Maisquellwasser, 6,0% Stärke, 0,5% $CaCO_3$ (pH-Einstellung vor Sterilisation auf 7,0 mit KOH, Sterilisation 30 Min. 121°) enthalten, und bebrütet diese Kultur bei 28° auf einer Rundschüttelmaschine, so erhält man nach einer Kulturdauer von 5 Tagen eine Kulturbrühe, deren Gehalt an Wirkstoff gemäß Beispiel 17 zu 2,8 mg/Ltr. bestimmt wird.

## Beispiel 2

Beimpft man einen Fermenter, der 100 Ltr. einer Nährlösung nach Beispiel 1 enthält, mit 3 Ltr. einer in Schüttelkolben gewonnenen Vorkultur des Stammes SS 237 und bebrütet unter Rührung und

**0 002 695**

Belüftung 6 Tage bei 28°, so erhält man eine Kulturbrühe, aus der nach Adsorption an einem hydrophoben Trägerharz auf Sulfonsäurebasis, z. B. Lewapol (Säule 30 × 50 cm, Fließrate 20 Ltr./Std.) und Desorption mit 50 Ltr. 50%igem Methanol (Fließrate 20 Ltr./Std.) im Lyophilisat des Desorbats ein Präparat gewonnen wird, das im Herbizidtest an Kresse bei einer Aufwandmenge von 1,5 kg/ha im pre-emergence-Test gemäß Beispiel 20 80%ige Abtötung ergibt.

### Beispiel 3

Beimpft man einen 1-Ltr.-Erlenmeyerkolben, der 120 ml einer Nährlösung aus 2% Stärke, 1% Glucose, 0,5% Kaseinhydrolysat, 1,0% Hefeextrakt enthält, pH mit $Na_2CO_3$ auf 7,2 eingestellt und dann mit 0,4% $CaCO_3$ versetzt und 30 Min. bei 121° sterilisiert, mit 8 ml einer Vorkultur des Stammes SS 243, gewonnen in derselben Nährlösung, und bebrütet auf einer Rundschüttelmaschine, so erhält man nach 6 Tagen eine Kulturbrühe, deren Gehalt an Wirkstoff gemäß Beispiel 17 zu 2,8 mg/Ltr. bestimmt wird.

### Beispiel 4

Beimpft man einen Ansatz nach Beispiel 3 mit einer Vorkultur des Stammes SS 248, so erhält man nach einer 6-tätigen Bebrütung bei 28° eine Kulturbrühe, deren Gehalt an Wirkstoff gemäß Beispiel 17 zu 4,7 mg/Ltr. bestimmt wird.

### Beispiel 5

Beimpft man einen 1-Ltr.-Erlenmeyerkolben, der 120 ml einer Nährlösung, bestehend aus 1% Glucose, 1% Stärke, 0,5% Kaseinhydrolysat, 0,75% Fleischextrakt, 0,75% Pepton, 0,5% Hefeextrakt, 0,1% $K_2HPO_4$, 0,3% NaCl, 0,1% $MgSO_4$, pH vor der Sterilisation auf 7,2 eingestellt, 30 Min. bei 121° sterilisiert, enthält, mit 8 ml einer Vorkultur des Stammes SS 268, gewonnen in derselben Kulturlösung und bebrütet auf einer Rundschüttelmaschine 6 Tage bei 28°, so erhält man eine Kulturbrühe, deren Gehalt an Wirkstoff gemäß Beispiel 17 zu 23,8 mg/Ltr. bestimmt wird.

### Beispiel 6

Arbeitet man nach Beispiel 5, jedoch mit einer Nährlösung ohne Glucose, so erhält man nach 3tägiger Bebrütung eine Kulturbrühe, deren Gehalt an Wirkstoff gemäß Beispiel 17 zu 24,6 mg/Ltr. bestimmt wird.

### Beispiel 7

Arbeitet man nach Beispiel 6, jedoch bei 24°, so erhält man nach 4tätiger Bebrütung bei einer Gehaltsbestimmung gemäß Beispiel 17 24,1 mg Wirkstoff je Ltr.

### Beispiel 8

Ersetzt man in Beispiel 5 die Glucose durch Stärke, so erhält man nach 4 Tagen bei einer Gehaltsbestimmung gemäß Beispiel 17 29,5 mg Wirkstoff je Ltr.

### Beispiel 9

Ersetzt man in Beispiel 5 Glucose durch Saccharose, so erhält man nach 4 Tagen bei einer Gehaltsbestimmung gemäß Beispiel 17 30,8 mg Wirkstoff je Ltr.

### Beispiel 10

Ersetzt man in Beispiel 5 Glucose durch Galactose, so erhält man nach 5 Tagen bei einer Gehaltsbestimmung gemäß Beispiel 17 38,3 mg Wirkstoff je Ltr.

13

# 0 002 695

## Beispiel 11

Arbeitet man nach Beispiel 5, jedoch ohne Kaseinhydrolysat, so erhält man nach 5tägiger Bebrütung bei einer Gehaltsbestimmung gemäß Beispiel 17 13,4 mg Wirkstoff je Ltr.

## Beispiel 12

Arbeitet man nach Beispiel 5, jedoch mit 1% Kaseinhydrolysat, so erhält man nach 5tägiger Bebrütung bei einer Gehaltsbestimmung gemäß Beispiel 17 10,5 mg Wirkstoff je Ltr.

## Beispiel 13

Arbeitet man nach Beispiel 5, jedoch ohne Fleischextrakt, so erhält man nach 6tägiger Bebrütung bei einer Gehaltsbestimmung gemäß Beispiel 17 13,1 mg Wirkstoff je Ltr.

## Beispiel 14

Arbeitet man nach Beispiel 5, jedoch mit 1,5% Fleischextrakt, so erhält man nach 5tägiger Bebrütung bei einer Gehaltsbestimmung gemäß Beispiel 17 14,5 mg Wirkstoff je Ltr.

## Beispiel 15

Bei einer Fermentation nach Beispiel 2 werden nach dem Abzentrifugieren des Mycels 85 Ltr. Kulturüberstand gewonnen.

Der Kulturüberstand wurde mit 20 Ltr./Std. auf eine Säule ($30 \times 50$ cm) die ein hydrophobes Trägerharz auf Sulfonsäurebasis (z. B. Lewapol) enthält, aufgetragen und die Säule mit 50 Ltr. $H_2O$ nachgewaschen. Durchlauf und Waschwasser waren unwirksam und wurden verworfen. Anschließend wurde mit 50 Ltr. 50%igem Methanol desorbiert, das Desorbat im Rotationsverdampfer auf ca. 2 Ltr. eingeengt und lyophilisiert (Ausbeute: 1039 g). Zur Vervollständigung der Desorption wurde die Säulenfüllung mit 50 Ltr. 100%igem Methanol nachgewaschen, das Eluat zur Trockne eingeengt, der so erhaltene Rückstand in 2 Ltr. Wasser aufgenommen und die Suspension lyophilisiert (Ausbeute: 20,4 g) Das Desorbat vom Trägerharz mit 50%igem Methanol zeigte die herbizide Wirkung.

Die Lösung von 150 g des 50%-Desorbats von Lewapol in 20 Ltr. 0,01 M Ammoniumformiat-Puffer pH 3,6 wurde mit 1 kg Phosphocellulose ($H^+$-Form) versetzt. Das Gemisch wurde 1 Std. gerührt und danach über eine Nutsche (Filter K3) abgesaugt, der eingeengte Durchlauf wurde lyophilisiert (Ausbeute: 89,1 g). Das Lyophilisat war im Herbizid-Test unwirksam. Die Phosphocellulose wurde in 10 Ltr. 1 M Ammoniumformiat-Lösung eingegeben, das Gemisch 1 Std. gerührt und erneut abgesaugt. Das Lyophilisat des Durchlaufs (1,53 g) enthielt die herbizide Wirkkomponente.

1,5 g des Ammoniumformiatdesorbats der Phosphocellulose wurden mit 20 ml $10^{-2}$ M Ammoniumformiat-Lösung behandelt, der unlösliche Anteil wurde abzentrifugiert (Rückstand unwirksam) und der Überstand auf eine Biogel P-2-Säule ($5 \times 100$ cm, äquilibriert mit $10^{-2}$ M Ammoniumformiat-Lösung) aufgetragen. Das Eluat wurde in 6 Fraktionen aufgetrennt, Fraktion D zeigte die herbizide Wirkung (Ausbeute: 74 mg).

Die Fraktion der Trennung an Biogel® P-2 (Polyacrylamidgel der Fa. Bio-Rad) wurde dünnschichtchromatographisch auf Reinheit geprüft. Es zeigte sich, daß die Fraktion noch nicht chemisch einheitlich war. Daher wurde die Lösung von 70 mg der Fraktion D in 2 ml Chloroform/Methanol = 4/1 durch eine präparative Papierchromatographie fraktioniert (Papier: Whatman 3 mm, Laufmittel Chloroform/Methanol = 4/1). Vier Zonen wurden mit Wasser eluiert. Die zweite Zone ($R_f = 0,05 - 0,12$) enthielt den herbiziden Wirkstoff (26 mg).

## Beispiel 16

Adsorbiert man 85 Ltr. eines Kulturfiltrats gemäß Beispiel 2 an einem hydrophoben Trägerharz auf Sulfonsäurebasis, z. B. Lewapol, (Säule $30 \times 50$ cm, Fließrate 20 Ltr./Std.) und desorbiert dann mit 50 Ltr. 50%igem Methanol, so erhält man nach anschließendem Lyophilisieren 147 g Rohpräparat. 5 g dieses Rohpräparates können einer präparativen Hochdruckflüssigkeitschromatographie an Kieselgel (Fertigsäule zum präparativen HPLC, System LC 500, Fa. Waters) in Chloroform/Methanol im Volumenverhältnis 1/1 unterworfen werden. Hierzu wird eine Lösung von 5 g 50% methanolisches Desorbat von Lewapol in 50 ml Laufmittel gelöst und zur Entwicklung eingespritzt. Die Fließrate beträgt dabei 250 ml/Std. Unter diesen Bedingungen beträgt die Retentionszeit des Wirkstoffs 7 Min.

14

**0 002 695**

20 Sek. Nach dem Lyophilisieren erhält man 379 mg weißes amorphes Pulver, das gemäß Beispiel 20 mit einer Aufwandmenge von 1,25 kg/ha wirksam ist.

### Beispiel 17

10 ml eines Kulturfiltrats gemäß Beispiel 3 werden mit 100 µl 1 M Acetatpuffer pH 3,5 versetzt. Es wird 30 Min. gerührt und dann abzentrifugiert. Zum Überstand werden 500 mg Phosphocellulose ($H^+$-Form) zugegeben. Die Suspension wird 10 Min. gerührt, anschließend wird abzentrifugiert. Das Herbizid wird hierbei an den Ionenaustauscher gebunden. Zum Sediment (Phosphocellulose) werden 5 ml 1 N Ammoniumformiatlösung zugegeben, es wird erneut 10 Min. gerührt und dann abzentrifugiert. Der Überstand wird am Rotationsverdampfer zur Trockne eingeengt. Um den Salzgehalt der anschließend zur Dünnschichtchromatographie verwendeten Lösung gering zu halten, wird der Rückstand in 1 ml 90%igem Methanol aufgenommen. Es wird erneut abzentrifugiert und 10 µl des Überstandes zur Dünnschichtchromatographie eingesetzt (Lösung I). Die Chromatographie erfolgt auf silanisierten Kieselgelplatten mit Fluoreszenzindikator. Die Entwicklung erfolgt im Laufmittel Chloroform/Methanol/0,01 M Tris/HCl-Puffer pH 3,0 (Volumenverhältnis 4/1/0,02). Es wird über 15 cm entwickelt. Das Profil der Fluoreszenzlöschung wird an einem DC-Scanner der Fa. Carmag) aufgezeichnet (Bedingungen: 1-Punkt-Blende, Primärfilter 810, Sekundärfilter 2A+10% 823, Spaltbreite 5 mm). Das Herbizid besitzt einen $R_f$-Wert von 0,26.

Je 10 µl Lösung I werden mit dem Herbizid gemäß Beispiel 15 als Vergleichssubstanz auf Kieselgel 60, F 254 (Fa. Merck) chromatographiert. Als Fließmittel dienen:

1) n-Butanol/Eisessig/$H_2O$ = 50/25/25
2) iso-Propanol/2 N Ammoniak/$H_2O$ = 70/10/20
3) n-Butylacetat/n-Butanol/Eisessig/Phosphatpuffer pH 7 = 50/10/25/15

Die Wirkstoffe gemäß den Beispielen 1 und 3 verhalten sich dünnschichtchromatographisch gleich (Laufmittel 1): $R_f$ = 0,27, Laufmittel 2): $R_f$ = 0,40 und Laufmittel 3): $R_f$ = 0,08).

Beide Wirkstoffe lassen sich mit 4-Dimethylaminobenzaldehyd, Ninhydrin und Morgan-Elson's-Reagens anfärben.

Die quantitative Bestimmung des Herbizids in den Kulturfiltraten gemäß Beispiel 1 bis 14 erfolgt nach der Aufarbeitung durch Scannen des entwickelten Dünnschichtchromatogramms. Dabei werden die aufgezeichneten Peaks ausgeschnitten und das Papiergewicht bestimmt. Für die Reinsubstanz wird für 50 µg ein Papiergewicht von ca. 28 mg gefunden.

### Beispiel 18

5 ml der Fraktion D gemäß Beispiel 15 werden in einer präparativen Dünnschichtchromatographie an Kieselgel eingesetzt (Kieselgel 60, 2,5 mm, Fa. Merck). Als Laufmittel dient Chloroform/Methanol im Volumenverhältnis 80/20. Es wird über 15 cm entwickelt. Die Zone, die 1,5 cm bis 2,25 cm vom Start entfernt ist, wird ausgekratzt, mit 5 ml Wasser eluiert und lyophilisiert. Man erhält 12,5 mg reinen Wirkstoff.

### Beispiel 19

5 ml der Fraktion D gemäß Beispiel 15 werden in einer präparativen Papierchromatographie (Papier 3 mm, Fa. Whatman) eingesetzt. Als Laufmittel dient Chloroform/Methanol = 4/1. Es wird über 30 cm entwickelt. Die Zone, die 1,5 cm bis 3,6 cm von der Startlinie entfernt ist, wird ausgeschnitten und das Papier zweimal mit je 10 ml Wasser eluiert. Nach dem Lyophilisieren erhält man 14,1 mg reinen Wirkstoff.

### Beispiel 20

#### pre-emergence-Test

In Schalen, die mit Vermiculite gefüllt sind, werden Samen von Kresse (Lepidium sativum) ausgelegt. Die Schalen werden dann mit einer Hoagland-Nährlösung gegossen, der die erfindungsgemäßen mikrobiellen Wirkstoffe in den angegebenen Mengen zugesetzt sind.

Nach 2 Wochen wird der Schädigungsgrad der Pflanzen bonitiert in %-Schäden im Vergleich zur Entwicklung der unbehandelten Kontrolle. Es bedeuten:

15

0 002 695

0% = keine Wirkung (wie unbehandelte Kontrolle)
100% = totale Vernichtung

Wirkstoffe, Aufwandmengen und Resultate gehen aus der nachfolgenden Tabelle hervor.

Tabelle A

pre-emergence-Test

| Wirkstoffe | Dosierung, kg/ha | % Wirkung gegen Kresse |
|---|---|---|
| Präparat gemäß Beispiel 15 (Reinsubstanz) | 1,25 | 90 |
| Präparat gemäß Beispiel 3 und 15 (Lewapoldesorbat) (gereinigte Substanz) | 1,25 | 90 |
| Präparat gemäß Beispiel 4 und 15 (Lewapoldesorbat) (Rohsubstanz) | 40 | 70 |
| Präparat gemäß Beispiel 5 (Rohsubstanz) | 40 | 99 |

Beispiel 21

post-emergence-Test

In Schalen, die mit Vermiculite gefüllt sind, werden Samen von Kresse (Lepidium sativum) ausgelegt. Die Schalen werden dann mit einer Hoagland-Nährlösung gegossen, bis die Pflanzen eine Größe von 5 – 10 cm erreicht haben.

Dann werden die Pflanzen mit einer Wirkstoffzubereitung so gespritzt, daß die in der Tabelle angegebenen Dosierungen ausgebracht werden.

Nach 2 Wochen wird der Schädigungsgrad der Pflanzen boniert in % Schädigung im Vergleich zur Entwicklung der unbehandelten Kontrolle. Es bedeuten:

0% = keine Wirkung (wie unbehandelte Kontrolle)
100% = totale Vernichtung

Wirkstoffe, Aufwandmengen und Resultate gehen aus der nachfolgenden Tabelle hervor.

Tabelle B

post-emergence-Test

| Wirkstoffe | Dosierung, kg/ha | % Wirkung gegen Kresse |
|---|---|---|
| Präparat gemäß Beispiel 15 (Reinsubstanz) | 1,25 | 98 |
| Präparat gemäß Beispiel 3 und 15 (Lewapoldesorbat) (gereinigte Substanz) | 1,25 | 90 |
| Präparat gemäß Beispiel 4 und 15 (Lewapoldesorbat) (Rohsubstanz) | 40 | 60 |
| Präparat gemäß Beispiel 5 (Rohsubstanz) | 40 | 100 |

**Patentansprüche**

1. Verbindung gekennzeichnet durch die Summenformel $C_{10}H_{14}N_6O_3$, einen Zersetzungspunkt von 134 bis 135°C, eine spezifische Drehung von $[\alpha]_D^{20} = -10,7°$ gemessen in 1%iger Methanollösung und ein Molekulargewicht von 266.

16

2. Verfahren zur Herstellung einer Verbindung gekennzeichnet durch die Summenformel C₁₀H₁₄N₆O₃, einen Zersetzungspunkt von 134 bis 135°C, eine spezifische Drehung von $[\alpha]_D^{20} = -10{,}7°$ gemessen in 1%iger Methanollösung und ein Molekulargewicht von 266, dadurch gekennzeichnet, daß man einen Streptosporangium-Stamm aus der Reihe SS 237 (CBS 545.77), SS 243 (CBS 544.77), SS 248 (CBS 543.77), SS 268 (CBS 542.77) in einem Nährmedium, welches assimilierbare Kohlenstoff und Stickstoffquellen sowie Mineralsalze enthält, unter aeroben Bedingungen kultiviert und die Verbindung isoliert.

3. Herbizides Mittel enthaltend eine Verbindung gemäß Anspruch 1 oder 2.

4. Verwendung einer Verbindung gemäß Anspruch 1 oder 2 zur Bekämpfung von unerwünschtem Pflanzenwachstum.

5. Streptosporangium-Stämme SS 237 (CBS 545.77), SS 243 (CBS 544.77), SS 248 (CBS 543.77) und SS 268 (CBS 542.77).

## Claims

1. A compound characterised by the empirical formula C₁₀H₁₄N₆O₃, a decomposition point of 134 to 135°C, a specific rotation of $[\alpha]_D^{20} = -10.7°$ measured in a 1% methanol solution and a molecular weight of 266.

2. Process for the preparation of a compound characterised by the empirical formula C₁₀H₁₄N₆O₃, a decomposition point of 134 to 135°C, a specific rotation of $[\alpha]_D^{20} = -10.7°$ measured in a 1% methanol solution and a molecular weight of 266, characterised in that a Streptosporangium strain from the series SS 237 (CBS 545.77), SS 243 (CBS 544.77), SS 248 (CBS 543.77) and SS 268 (CBS 542.77) is cultured under aerobic conditions in a nutrient medium which contains assimilable carbon and nitrogen sources as well as mineral salts, and the compound is isolated.

3. A herbicidal composition containing a compound according to claim 1 or 2.

4. Use of a compound according to claim 1 oder 2 for combating undesired plant growth.

5. The Streptosporangium strains SS 237 (CBS 545.77), SS 243 (CBS 544.77), SS 248 (CBS 543.77 and SS 268 (CBS 542.77).

## Revendications

1. Composé, caractérisé par la formule brute C₁₀H₁₄N₆O₃, un point de décomposition de 134 à 135°C, une rotation spécifique $[\alpha]_D^{20} = -10{,}7°$ mesurée sur une solution méthanolique à 1% et un poids moléculaire de 266.

2. Procédé de production d'un composé, caractérisé par la formule brute C₁₀H₁₄N₆O₃, un point de décomposition de 134 à 135°C, une rotation spécifique $[\alpha]_D^{20} = -10{,}7°$ mesurée sur une solution méthanolique à 1% et un poids moléculaire de 266, caractérisé en ce qu'on cultive dans des conditions aérobies une souche de Streptosporangium de la série SS 237 (CBS 545.77), SS 243 (CBS 544.77), SS 248 (CBS 543.77), SS 268 (CBS 542.77) dans un milieu nutritif qui contient des sources de carbone et d'azote assimilables ainsi que des sels minéraux et on isole le composé.

3. Une composition herbicide contenant un composé suivant la revendication 1 ou 2.

4. Utilisation d'un composé suivant la revendication 1 ou 2 pour la lutte contre la croissance indésirable de plantes.

5. Les souches de Streptosporangium SS 237 (CBS 545.77), SS 243 (CBS 544.77), SS 248 (CBS 543.77) et SS 268 (CBS 542.77).

# FIG. 1

## FIG. 2

# FIG. 3

# FIG.4